# EUROPEAN PATENT SPECIFICATION

(11) **EP 2 783 011 B1**
(45) Date of publication and mention of the grant of the patent: **21.02.2018**
(21) Application number: 12809298.8
(22) Date of filing: 22.11.2012
(51) Int. Cl.: C12Q 1/04, G01N 33/574, G01N 33/68, A61B 10/02, A61B 10/04

(54) **METHODS FOR DETECTING OF BENIGN CONDITIONS**
VERFAHREN ZUM NACHWEIS BENIGNE ERKRANKUNGEN
MÉTHODES DE DÉTECTION D'AFFECTIONS BÉNIGNES

(30) Priority: 22.11.2011 GB 201120162
(43) Date of publication of application: 01.10.2014
(73) Proprietor: Medical Research Council, Swindon SN2 1FL (GB)
(72) Inventor: FITZGERALD, Rebecca C., CambridgeCB2 2XZ (GB); O'DONOVAN, Maria, Cambridge (GB)
(74) Representative: Soames, Candida Jane
(86) International application number: PCT/GB2012/000855
(87) International publication number: WO 2013/076444

(56) References cited:
- WO-A1-2007/045896
- WO-A1-2011/058316
- US-A- 5 420 016
- US-A1- 2007 178 510
- US-A1- 2012 009 597
- LAO-SIRIEIX, P., ET AL: "Non-endoscopic screening biomarkers for Barrett's oesophagus: from microarray analysis to the clinic", GUT, vol. 58, 2009, pages 1451-1459, XP009167512,
- ANIM J T ET AL: "Assessment of different methods for staining Helicobacter pylori in endoscopic gastric biopsies", ACTA HISTOCHEMICA, ELSEVIER, AMSTERDAM, NL, vol. 102, no. 2, 1 January 2000 (2000-01-01), pages 129-137, XP004631861, ISSN: 0065-1281, DOI: 10.1078/S0065-1281(04)70022-7

## Description

### Field of the Invention

The invention relates to a method for aiding the diagnosis of an inflammatory condition of the upper gastrointestinal tract. In particular, the invention relates to the use of a surface sampling technique in new medical applications to benign conditions of the oesophagus.

### Background to the invention

Symptoms of oesophageal disease which include heartburn, dyspepsia (indigestion), dysphagia (difficulty in swallowing) and odynophagia (pain on swallowing) are common causes for attendance at GP surgeries. The symptoms are not in themselves diagnostic for specific oesophageal diseases and therefore in order to reach a diagnosis referral for endoscopy is required. Endoscopy is invasive, costly and requires patients to attend a specialist centre and take time off work. As an alternative the NICE guidelines therefore encourage GP's to treat empirically with acid-suppressant drugs unless there are so called alarm symptoms (http://guidance.nice.org.uk/CG17). These drugs are expensive, can cause side-effects and the patient is left without a specific diagnosis.
When patients are referred for endoscopy oesophageal and gastric sampling is required to confirm the diagnosis. These samples may be directed towards a visible abnormality but may also be taken at random to diagnose inconspicuous diagnoses such as eosinophilic oesophagitis for example. Hence, multiple biopsies are recommended which has inherent risk and there is also sampling bias which can lead to false negative results. These endoscopic samples are usually biopsies examined by histopathology but to diagnose infections oesophageal brushings may be taken which are processed to a monolayer. Therefore multiple samples may be taken at the same procedure.
The commonest gastric and duodenal conditions are associated with *H.pylori* infection which can lead to peptic ulcers and predisposes to gastric cancer [4]. Eosinophilic esophagitis has been increasing in the west in line with increasing allergic conditions such as asthma hay fever, allergic rhinitis and atopic dermatitis. If occurs in approximately 0.4% of the population but increases to 4% of those with refractory reflux symptoms and is an important cause of food bolus obstruction [5].

Cell collection devices such as a cytosponge have been applied in detection of cancers. A swallowable cell collection device is described in WO2007/045896. This document discloses kits and methods for aiding the diagnosis of Barrett's oesophagus or Barrett's associated dysplasia. In particular, this document teaches the collection of cells which are then subjected to molecular tests for markers of pre-malignant or cancerous cells within the oesophagus. This is accomplished by detection of various specific molecular biomarkers which are indicative of malignant or dysplastic cells.

WO2011/058316 discloses a swallowable cell sampling device. The device comprises an abrasive material capable of collecting cells from the surface of the oesophagus, and a means for retrieval of the device. This document discloses that the means for retrieval comprises a cord which is attached to the abrasive material by means of a specific type of knot (a hitch knot). The focus of this patent application is the device itself and the technical benefits provided by the means of attachment of the device to the cord for retrieval. Various applications of this device are disclosed, which are consistently targeted towards dysplasia and cancerous conditions such as adenocarcinoma.

*Candida albicans* infections of the oesophagus can be detected according to prior art techniques. These prior art techniques involve collection of endoscopic brushings. These brushing contain cells. These cells are then prepared into a monolayer, and the resulting monolayer forms the basis of the test for *C.albicans* infection.

*Helicobacter pylori* infection of the upper *gastrointestinal* tract can be detected according to prior art techniques. These involve taking biopsies from the stomach which then form the basis of the test. Alternatively, *H.pylori* infection can be inferred by taking a blood sample from the patient and testing for particular antibodies which react with *H.pylori* surface proteins.

Oesophagitis can be detected by prior art techniques. These involve the use of an endoscopy to grade the oesophagitis. At this stage the endoscopist has to take the decision whether or not biopsies are also required. The diagnosis of oesophagitis is dependent on the skill and attention of the endoscopist.

Eosinophilic oesophagitis is a condition linked to allergic or asthmatic sensitivity. Eosinophilic oesophagitis appears normal by endoscopy. Therefore, the standard procedure for diagnosing the eosinophilic oesophagitis is to take multiple biopsies. Typically six such biopsies are taken - two from the top, two from the middle and two from the lower regions of the oesophagus. This is clearly an extended and invasive procedure. This requires considerable operator time and skill for a highly trained endoscopist. This also involves considerable patient discomfort.

The present invention seeks to overcome problems associated with the prior art.

### Summary of the invention

The present invention is defined in independent claim 1 and certain optional features thereof are defined in the dependent claims. In so far as the terms "invention", "example" and "embodiment" are used herein, this will be interpreted in such a way that the only protection sought is for the invention as claimed. Cell sampling devices such as the cytosponge are known for detection of cancer. More specifically, cell collection devices such as the cytosponge have been used for detection of adenocarcinoma. These have also been used for detection of early stage or pre-malignant conditions such as dysplasia, or Barrett's oesophagus, which clinically can be regarded as early stage cancer lesions.

By contrast, the present inventors have realised that oesophagal cell collection can advantageously be used in the detection of an infection or inflammatory disorder. In particular, the present inventors have applied cell collection devices such as the cytosponge to benign conditions.

In more detail, the present inventors have realised that the presence of certain foreign cells in the oesophagus can be diagnostic for certain benign medical conditions. Therefore, the inventors have provided a new method for diagnosing benign conditions by assaying foreign cells collected using a cell collection device such as a cytosponge.

Thus, in one aspect the invention provides a method for detecting a foreign cell in a tissue of the upper intestinal tract, said method comprising:
(a) providing a sample of cells from the tissue of interest;
(b) preparing the cells into a cohesive cluster;
(c) staining cells from the cohesive cluster using haematoxylin and eosin (H&E) stain;
(d) observing the stained cells; and
(e) inferring the presence or absence of foreign cell(s) from the observations of (d).

In another aspect the invention provides a method of collecting information useful for detecting a foreign cell in a tissue of the upper intestinal tract, said method comprising:
(a) providing a sample of cells from the tissue of interest;
(b) preparing the cells into a cohesive cluster;
(c) staining cells from the cohesive cluster using haematoxylin and eosin (H&E) stain;
(d) observing the stained cells; and
(e) inferring the presence or absence of foreign cell(s) from the observations of (d).

In one aspect the invention provides a method for detecting an infection in a tissue of the upper intestinal tract, or a method of collecting information useful for detecting an infection in a tissue of the upper intestinal tract, said method comprising:
(a) providing a sample of cells from the tissue of interest;
(b) preparing the cells into a cohesive cluster;
(c) staining cells from the cohesive cluster using haematoxylin and eosin (H&E) stain;
(d) observing the stained cells; and
(e) inferring the presence or absence of infection from the observations of (d), for example the observation of foreign cell(s) of the infective organism (eg. Candida, Aspergillus, H.pylori) or cells infected by the infective organism (eg. Herpes) infer infection by that organism.

The prior art techniques always involve production of monolayers from sampled cells. By contrast the inventors teach preparing the cells into a cohesive cluster, for example by clotting the cells. This has the advantage of avoiding problems of overlapping cells which hinder prior art monolayer approaches. This has the advantage of requiring only a single section (e.g. a single slide) to be prepared. This has the advantage of minimising the labour involved in obtaining a representative sample (e.g. slice/section) for analysis.

Suitably the cells in the sample are from the epithelium. Suitably the cells comprise epithelial cells. Suitably the cells are obtained by sampling the epithelium.
Clearly, the cells sampled from the epithelium will not always consist exclusively of epithelial cells since foreign cells may be present, detection of which foreign cells contributes to diagnosis (or information useful for aiding diagnosis) as discussed herein. The important point is that it is the epithelial cells (epithelium layer) which are (is) sampled and therefore the sample will suitably comprise mostly or predominantly epithelial cells.
It is an advantage of the invention that epithelial cells are sampled because it is possible to observe inflammatory cells in deeper layers of normal tissues, such as in the lamina propria. It is an advantage of the invention that when the sample is collected using a swallowable device comprising abrasive material (such as a cytosponge), only the epithelium/epithelial cells are targeted. This provides the advantage that observation of foreign cell types such as inflammatory cells in (amongst) epithelial cells is more meaningful than if such cells were observed in (amongst) cells of the lamina propria (which is advantageously not sampled according to the present invention).

Thus more suitably the invention provides a method for detecting a foreign cell in a tissue of the upper intestinal tract, said method comprising:
(a) providing a sample of cells comprising epithelial cells from the tissue of interest;
(b) preparing the cells into a cohesive cluster;
(c) staining cells from the cohesive cluster using haematoxylin and eosin (H&E) stain;
(d) observing the stained cells; and
(e) inferring the presence or absence of foreign cell(s) from the observations of (d).

Thus more suitably the invention provides a method for detecting a foreign cell in a tissue of the upper intestinal tract, said method comprising:
(a) providing a sample of cells from the epithelium layer of the tissue of interest;
(b) preparing the cells into a cohesive cluster;
(c) staining cells from the cohesive cluster using haematoxylin and eosin (H&E) stain;
(d) observing the stained cells; and
(e) inferring the presence or absence of foreign cell(s) from the observations of (d).

In another aspect, the invention relates to a method for aiding the diagnosis of an inflammatory condition of the upper intestinal tract in a subject, said method comprising detecting a foreign cell as described above, wherein detection of foreign cell(s) in step (e) infers that the subject has an inflammatory condition of the upper intestinal tract.

In another aspect the invention provides a method of collecting information useful for aiding the diagnosis of an inflammatory condition of the upper intestinal tract in a subject, said method comprising detecting a foreign cell as described above, wherein detection of foreign cell(s) in step (e) infers that the subject has an inflammatory condition of the upper intestinal tract.

In another aspect, the invention relates to a method as described above wherein the cohesive cluster is prepared by combining the cells with a sample of plasma and inducing said mixture to clot.

Suitably said sample of cells consists of cells collected from the upper intestinal tract, most suitably the surface of the upper intestinal tract.

The upper intestinal tract may include gastric cardia, gastro-oesophagal junction and oesophagus. Suitably the tissue is oesophagus. Suitably the sample is from the oesophagus.

Suitably said cells are cells collected using a swallowable device comprising abrasive material capable of collecting cells from the surface of the oesophagus. Thus a cytosponge may be defined as a swallowable device comprising abrasive material capable of collecting cells from the surface of the oesophagus.

Suitably said device is a capsule sponge (a type of cytosponge where the abrasive material is compressed into a capsule for ease of swallowing).

Suitably detection of foreign cell(s) infers that the subject has a disorder of the upper gastrointestinal tract, said method further comprising classifying the foreign cell(s) observed in step (d), and inferring the type of disorder based on the type(s) of cell(s) observed.

Suitably if the foreign cell is a leukocyte, it is inferred that the subject has oesophagitis;
if the foreign cell is a neutrophil, it is inferred that the subject has oesophagitis;
if the foreign cell is a eosinophil, it is inferred that the subject has eosinophilic oesophagitis;
if the foreign cell is a lymphocyte, it is inferred that the subject has lymphocytic gastritis; if the foreign cell is *H.pylori,* it is inferred that the subject has *H.pylori* infection; and
if the foreign cell is *C.albicans,* it is inferred that the subject has *C.albicans* infection.

Suitably if the foreign cell is a leukocyte, it is inferred that the subject has oesophagitis;
if the foreign cell is a neutrophil, it is inferred that the subject has oesophagitis;
if the foreign cell is a eosinophil, it is inferred that the subject has eosinophilic oesophagitis;
if the foreign cell is a lymphocyte, it is inferred that the subject has lymphocytic gastritis;
if the foreign cell is *H.pylori,* it is inferred that the subject has *H.pylori* infection;
if the foreign cell is *C.albicans,* it is inferred that the subject has *C.albicans* infection;
if the foreign cell is *Aspergillus,* it is inferred that the subject has *Aspergillus* infection; and
if the foreign cell is a cell infected with Herpes virus, it is inferred that the subject has Herpes infection.

In another aspect, the invention relates to a method as described above further comprising performing a Giemsa stain.

In another aspect, the invention relates to a method as described above further comprising testing for urease activity.

In another aspect, the invention relates to a method as described above further comprising staining using an antibody reactive against *H.pylori.*

In another aspect, the invention relates to a cytosponge for use in detection of a foreign cell in a tissue of the upper gastrointestinal tract, such as in the epithelium layer of said tissue.
In another aspect, the invention relates to a cytosponge for collecting information useful for detection of a foreign cell in a tissue of the upper gastrointestinal tract, such as in the epithelium layer of said tissue.

In another aspect, the invention relates to a cytosponge as described above wherein the foreign cell is selected from the group consisting of: leukocyte, neutrophil, eosinophil, lymphocyte, *H.pylori,* and *C.albicans,*

In another aspect, the invention relates to a cytosponge as described above wherein the foreign cell is selected from the group consisting of: leukocyte, neutrophil, eosinophil, lymphocyte, *H.pylori, C.albicans,* Aspergillus, and a cell infected with Herpes virus.

In another aspect, the invention relates to a cytosponge as described above wherein the tissue of the upper gastrointestinal tract is oesophagus.

In another aspect, the invention relates to a cytosponge for use in diagnosis of a benign condition of the upper gastrointestinal tract.
In another aspect, the invention relates to a cytosponge for collecting information useful for diagnosis of a benign condition of the upper gastrointestinal tract.
In another aspect, the invention relates to a cytosponge as described above wherein the benign condition is an inflammatory condition.
In another aspect, the invention relates to a cytosponge as described above wherein the inflammatory condition is *H.pylori* infection, *C.albicans* infection, oesophagitis, lymphocytic gastritis, or eosinophilic oesophagitis.
In another aspect, the invention relates to a cytosponge as described above wherein the inflammatory condition is *H.pylori* infection, *C.albicans* infection, oesophagitis, lymphocytic gastritis, or eosinophilic oesophagitis, *Aspergillus* infection, or Herpes virus infection.

In another aspect, the invention relates to a cytosponge for use in detection of a foreign cell in a tissue of the upper gastrointestinal tract, wherein detection is according to a method as described above.

In another aspect, the invention relates to a cytosponge for use in detection of a foreign cell in a tissue of the upper gastrointestinal tract,
wherein detection comprises the steps of
(a) providing a sample of cells comprising epithelial cells or a sample of cells from the epithelium layer of the tissue of interest;
(b) preparing the cells into a cohesive cluster;
(c) staining cells from the cohesive cluster using haematoxylin and eosin (H&E) stain.

In another aspect, the invention relates to a cytosponge for use in diagnosis of a benign condition of the upper gastrointestinal tract wherein diagnosis is according to a method as described above

In another aspect, the invention relates to a cytosponge for use in diagnosis of a benign condition of the upper gastrointestinal tract,
wherein diagnosis comprises the steps of
(a) providing a sample of cells comprising epithelial cells or a sample of cells from the epithelium layer of the tissue of interest;
(b) preparing the cells into a cohesive cluster;
(c) staining cells from the cohesive cluster using haematoxylin and eosin (H&E) stain.

### Detailed Description of the Invention

Benign conditions of the oesophagus are common and are either treated empirically with acid-suppressants or frequently when a diagnosis is warranted necessitate endoscopy. A non-endoscopic alternative is disclosed herein which can diagnose a range of conditions from a single sample. This has significant benefits for patients (less invasive and more acceptable than endoscopy) and GPs (cost-effective, rapid diagnostic information without referral to secondary care). The inventors have developed assays to diagnose a range of common oesophageal conditions (oesophagitis, eosinophilic oesophagitis and candidiasis) and *H.pylori* infection of the proximal stomach, and/or Aspergillus infection and/or Herpes virus infection, which can be applied to a single non-endoscopic test which is applicable to primary care.

In one aspect the invention may be viewed as a combination of the following elements
- providing a sample of cells from the upper intestinal tract such as oesophagus;
- processing those cells into a single sample for analysis;
- performing H & E stain on a single sample of those cells;
- inferring from observation of the stained cells the presence of any foreign cells.

Suitably the collected cells are processed into a single sample. This single sample can then be sectioned to provide a sample for analysis.

New uses of a non-endoscopic cell collection device such as a cytosponge advantageously allow for simultaneous diagnosis of a range of oesophago-gastric conditions without need for an endoscopy. The device suitably consists of a biocompatible sponge, contained within a gelatine capsule, which is attached to a string. The capsule is swallowed and dissolves within the stomach after 3-5 minutes. The sponge can then be retrieved by pulling on the string thus collecting a cytological specimen for analysis. The cytosponge procedure can be performed in the primary care setting with excellent tolerability [1, 2].

Thus, in the absence of alarm symptoms suggestive of cancer which need prompt referral for endoscopy, we teach that the cytosponge approach described could be a valuable substitute for endoscopy in individuals with symptoms caused by benign oesophago-gastric disease and would be a significant advantage to the GP and patient compared with empirical treatment in the absence of a specific diagnosis.

An immunocytological test was previously developed to diagnose Barrett's oesophagus but the inventors have now made the advance that samples collected in this manner may also be utilised to diagnose benign conditions including: inflammatory conditions oesophagitis and eosinophilic oesophagitis, as well as infections such as *Candida,* and in addition infections such as *Aspergillus,* and *Herpes* virus.

### Definitions

The term 'comprises' (comprise, comprising) should be understood to have its normal meaning in the art, i.e. that the stated feature or group of features is included, but that the term does not exclude any other stated feature or group of features from also being present.

Advantageously the invention avoids the preparation of a cell monolayer from the collected cells. Cell monolayers can have overlapping cells. Cell monolayers can be laborious to prepare. Cell monolayers can be expensive to maintain. By avoiding the preparation of a cell monolayer, the invention provides numerous advantages in simplifying the procedure, and/or reducing the cost, and/or reducing the labour involved.

The cells are suitably prepared into a single sample for staining.

The preparation of the collected cells into a single sample is ideally performed by the following steps:
- washing cells off the collection device;
- collecting the cells together, e.g. by centrifugation
- processing the collected cells e.g. by embedding in paraffin wax
- preparation of sample for analysis, e.g. by slicing a section through the collected cells embedded in paraffin wax.
In this embodiment suitably the single sample for analysis is the slice/section prepared from the collected paraffin embedded cells.

Suitably analysis is by staining such as H&E staining, followed by observation of the stained cells.

In order to facilitate the preparation of a single cell sample for analysis (such as paraffin wax embedded collected cells), cells may advantageously be prepared into a cohesive cluster.

Suitably a cohesive cluster is prepared by clotting, or by use of gels or gel like substances e.g. agar, for example suspension in soft agar. Most suitably clotting is used. To accomplish this, the collected cell pellet is typically mixed with a plasma sample, and then plasmin or thrombin or any other suitable clotting factor is then added. The plasma then clots around the collected cells. This clotted cell complex is much more tractable and more easily processed e.g. embedded into paraffin wax for slicing and final sample preparation.

In more detail,
- Plasma - thrombin: suitably reagents can be of any mammalian origin
- Agar clots: suitably any percentage solution
- Agarose clots: suitably any percentage solution

For Agar/Agarose clotting, an exemplary method is as follows:
- Centrifuge to collect cells (e.g. as per thrombin-plama clotting)
- Make a 1% solution of agar or agarose saline by warming up to melting temperature (40-100C)
- Cool solution down to 40C
- Add 200-400uL of agar or agarose solution to the centrifuged cells and resuspend well
- Process the samples for observation (e.g. as per the thrombin-plasma clotting)

Thus the preparation of the cells into a cohesive cluster is ideally performed by the following steps:
- washing cells off the collection device;
- collecting the cells together, e.g. by centrifugation
- suspending the collected cells in plasma
- adding clotting factor (e.g. plasmin, e.g. thrombin)
- incubating to allow clotting.
The cohesive cluster (which in this embodiment is the clotted cells) is then processed into a single sample for staining. Suitably this is carried out as described above, for example by the following steps:
- processing the clotted cells e.g. by embedding in paraffin wax
- preparation of sample for analysis, e.g. by slicing a section through the cohesive cluster (e.g. clotted cells) embedded in paraffin wax.

Thus in one embodiment suitably the cohesive cluster refers to clotted cells.

In the prior art, it is standard cytological practice to prepare biopsy cells into monolayers for analysis. This is entirely standard in the analysis of oesophageal biopsies. This is also standard practice in biopsies from (for example) gut, cervix and other biopsy tissues. By contrast, the present inventors have gone against the standard practice in the art and instead avoid the monolayer preparation step. Instead, the present inventors teach that the cells can be combined and sectioned for analysis by conventional microscopy. This is a stark contrast to prior art techniques which necessarily involve preparation of cell monolayers. Suitably the cells of the present invention are not prepared into a monolayer.

### Sample and Collection

Suitably the sample of cells is from the epithelium layer of the tissue of interest.

Suitably the sample of cells is collected using a swallowable cell collection device.

Suitably only the cellular surface of the upper intestinal tract such as the oesophagus of the subject is sampled. Suitably said sampling is not directed to a particular site within the oesophagus. Suitably only the surface of the oesophagus is sampled. This has the advantage of avoiding more invasive sampling techniques such as biopsy collection techniques which penetrate below the surface of the oesophagus.

Suitably the sample comprises cells from the surface of a subject's upper intestinal tract. Suitably the sample consists of cells from the surface of a subject's upper intestinal tract. Suitably the sample comprises cells from the surface of a subject's oesophagus. Suitably the sample consists of cells from the surface of a subject's oesophagus.

Suitably the sample is an in vitro sample.
Suitably the sample is an extracorporeal sample.

Suitably sampling the cellular surface of the upper intestinal tract such as the oesophagus comprises the steps of
(i) introducing a swallowable device comprising abrasive material capable of collecting cells from the surface of the oesophagus into the subject,
(ii) retrieving said device by withdrawal through the oesophagus, and
(iii) collecting the cells from the device.

Preferably step (i) comprises introducing a swallowable device comprising abrasive material capable of collecting cells from the surface of the oesophagus into the subject's stomach.

Suitably said cell collection device comprises a capsule sponge.

Suitably said cell collection device comprises withdrawal means such as string.

In a preferred embodiment, the invention involves the sampling of the cells from the surface of the oesophagus using a swallowable abrasive material, which material is retrieved from the patient and from which the cells are subsequently separated for analysis.

Suitably the majority of the surface of the oesophagus is sampled, more suitably substantially the entire surface of the oesophagus is sampled, preferably the entire surface. Suitably the whole internal surface of the oesophagus ie. the complete inner lumen is sampled.

By abrasive is meant that the material is capable of removing cells from the internal surface of the oesophagus. Clearly, since this is meant for use in a subject's oesophagus, 'abrasive' must be interpreted in the light of the application. In the context of the present invention the term 'abrasive' has the meaning given above, which can be tested by passing the material through the oesophagus in an appropriate amount/configuration and examining it to determine whether cells have been removed from the oesophagus.

Preferably the swallowable abrasive material is expandable. In this embodiment, preferably the abrasive material is of a smaller size when swallowed than when withdrawn. An expandable material may be simply a resilient material compressed such that when released from compression it will expand again back to a size approximating its uncompressed size. Alternatively it may be a material which expands eg. upon taking up aqueous fluid to a final size exceeding its original size.

In other words, preferably the material of the device expands, swells, inflates or otherwise increases in size between swallowing and withdrawal. Preferably the device is auto-expandable ie. does not require further intervention between swallowing and expansion. Preferably the device is not inflatable. Preferably the device expands by unfolding, unfurling, uncoiling or otherwise growing in size following removal of restraint after swallowing. Preferably the material of the device is compressible and reverts a size approximating its uncompressed size following swallowing. Preferably the device is constructed from a compressed material which is releasably restrained in a compressed state. Preferably the material is released from restraint after swallowing, allowing expansion of the device/material before withdrawal.

Suitably the device comprises compressible material which is compressed into capsule form. Suitably the compressible material is in the form of sponge material. Suitably the compressed sponge is at least partially surrounded by a soluble and/or digestible coat such as a capsule coat. Suitably the sponge is indigestible. Suitably the capsule coat is at least partially formed from gelatine. Suitably the capsule coat is fully formed from gelatine.

It may be desirable to make the whole device out of digestible material to increase safety in case of a device becoming lost in the subject. Naturally the abrasive material would need to be digested at a slower rate than the capsule and the cord would need to be similarly slowly digested. Suitably the abrasive material is non-digestible. Suitably the cord is non-digestible.

Suitably the abrasive material comprises polyurethane. more suitably polyurethane sponge.

Suitably the device is a capsule sponge. As will be apparent from the specification, a capsule sponge is a device comprising compressible sponge as the abrasive material, which sponge is compressed into a capsule shape, which capsule shaped compressed sponge is suitably reversibly restrained in its compressed state by at least a partial coat of soluble and/or digestible material such as gelatine. Suitably the device is a capsule sponge as described in WO2007/045896 and/or as described in WO2011/058316. These two documents describe the structure and/or construction of the cell collection devices (capsule sponges).

Suitably the sample does not comprise endoscopically collected material. Suitably the sample does not comprise endoscopic biopsy. Suitably the sample does not comprise endoscopic brushings.

Suitably the expanded (eg. decompressed) abrasive material of the device is approximately 3cm in the plane perpendicular to the axis of the oesophagus. Preferably this is the approximate diameter of the oesophageal lumen. More preferably this is slightly larger than the diameter of the oesophageal lumen, advantageously ensuring good contact with the inner surface of same as withdrawal/sampling takes place.

It is a feature of the invention that the sampling is not directed eg. visually directed to any particular part of the oesophagus. It is a further advantage of the invention that a greater proportion of the surface of the oesophagus is sampled than is achieved by prior art techniques such as endoscopic biopsy (which samples approximately 1% of the surface) or endoscopic brushing. Preferably at least 10% of the oesophageal surface is sampled, preferably at least 20%, preferably at least 30%, preferably at least 40%, preferably at least 50%, preferably at least 60 %, preferably at least 70%, preferably at least 80%, preferably at least 90%. In a most preferred embodiment, preferably substantially the entire oesophagus is sampled, preferably the whole inner lumen of the oesophagus is sampled. This applies equally to the in *vitro* sample even when the method of the invention does not include collection of the sample.

In one embodiment the swallowable cell sampling device comprises an abrasive material capable of collecting cells from the surface of the oesophagus, and a means for retrieval wherein the means for retrieval comprises a cord, the cord being attached to the abrasive material by means of a hitch knot. Suitably said hitch knot is a double overhand knot.

Suitably said abrasive material is compressible. Suitably said abrasive material comprises reticulated polyurethane.
Suitably said cord is attached to said abrasive material via a loop of cord arranged below the surface of the abrasive material, said loop being closed by the hitch knot.

Suitably said abrasive material is compressed and wherein said abrasive material is retained in a compressed state by a soluble capsule.
Suitably said soluble capsule comprises a gelatine capsule.
Suitably said capsule is capable of dissolution and the compressible abrasive material is capable of reverting to its uncompressed size within 5 minutes upon immersion in water at 30 degrees Celsius.

Suitably the device comprises an unswallowable element at the end distal from the swallowable abrasive material.

A preferred cell collection device is a 'capsule sponge' device where the abrasive material comprises a sponge or sponge like material such as polyurethane mesh and wherein said material is packed or compressed into a gelatine capsule for ease of swallowing. Thus the generic term 'sponge' or 'capsule sponge' is sometimes used to discuss the preferred cell collection device for ease of understanding but it will be apparent that other embodiments of the cell collection device are envisaged and that the invention is not to be understood as limited only to samples collected by the preferred capsule sponge embodiment(s).

The abrasive material suitably comprises a sponge or sponge like material.

In more detail, a key feature of the abrasive material is that the material needs to be abrasive enough to collect as many cells as possible whilst at the same time avoiding damage to the oesophagal lining. These advantageous features may be achieved for example via use of a sponge or honeycomb form of abrasive material. This porous or cavitated form of material maximises collection and/or entrapment of cells inside and on the surface of material. Moreover, the cavities or hollows in sponge like or honeycomb material such as reticulated polyurethane also facilitate compression which is advantageous in reducing the size of the material at administration for example via a soluble capsule.

Suitably the material has a uniform shape.
Suitably the material has a uniform diameter.

Suitably the uncompressed shape is round such as spherical.
Suitably the uncompressed diameter is 3cm.

Suitably the material is dimensioned to fit into a swallowable capsule such as a gelatine capsule, suitably in a compressed or stowed form. Suitably said material does not break the capsule whilst compressed inside, but only deforms or breaks the capsule once in use such as inside the subject.

Suitably the capsule has a uniform shape.

Suitably the capsule has a uniform size.

Suitably the capsule dissolves quickly such as dissolves in 30 degree centigrade water within 5 minutes.

Suitably said capsule is intact all over and does not have any breaks and sharp ends. This has the advantage of preventing injury while swallowing.

In one embodiment the method comprises the step of collecting cells using a swallowable cell collection device.

In practical terms the upper intestinal tract includes the gastric cardia (e.g. proximal stomach), the gastro-oesophagal junction (where the stomach meets the oesophagus) and the oesophagus itself. When the sample of cells is obtained using a swallowable device as described, cells from each of these three tissues are collected, such as cells from the epithelium layer of said tissues. This is because a swallowable cell collection device such as a cytosponge is swallowed, arrives in the stomach, and is then withdrawn in the collection step and so will contain cells from each of the three regions it contacts on its return journey. In the less common event that the cell sample contains only cells from the oesophagus i.e. when the cell sample consists of cells from the oesophagus then cells from the gastric cardia (proximal stomach) and gastro-oesophagal junction would not be present. In this situation *H.pylori* infection may be difficult to detect since *H.pylori* (when present) is normally resident in the proximal stomach (not the oesophagus). Thus when applied to diagnosis of *H.pylori* infection, suitably the sample comprises cells from the gastric cardia and/or gastro-oesophagal junction, most suitably cells from the gastric cardia.

In one embodiment suitably the method is an *in vitro* method. In one embodiment suitably the method is an extracorporeal method. In one embodiment suitably the actual sampling of the cells is not part of the method of the invention. Suitably the method does not involve collection of the cells. Suitably the sample is a sample previously collected. Suitably the method does not require the presence of the subject whose cells are being assayed. Suitably the sample is an in vitro sample. Suitably the method does not involve the actual medical decision, stricto sensu; such a decision stricto sensu would typically be taken by the physician.

Suitably the method of the invention is conducted in vitro. Suitably the method of the invention is conducted extracorporeally.

### Applications

The invention may be usefully applied to detection of foreign cells from the upper gastrointestinal tract. In particular, the invention may be applied to detection of foreign cells in the oesophagus. Moreover, the invention may be applied to the detection of foreign cells in the upper gastric cardia and/or oesophagus. More specifically, the invention may be applied to the detection of foreign cells in any one or more of the upper gastric cardia, gastro oesophageal junction and oesophagus. More suitably the invention is applied to the detection of foreign cells in any one or more of the gastro oesophageal junction and oesophagus. Most suitably the invention is applied to the detection of foreign cells in the oesophagus.

The method may be for detection of, or aiding diagnosis of, benign conditions of the upper gastrointestinal tract such as the oesophagus.

The invention can be applied to the detection of infections. For example, the invention may be applied to detection of *Candida albicans* infection. For example, the invention may be applied to detection of *Helicobacter pylori* infection. For example, the invention may be applied to detection of Aspergillus infection. For example, the invention may be applied to detection of Herpes infection.

The invention may be applied to detection of inflammatory conditions of the upper intestinal tract such as the oesophagus. For example, the invention may be applied to detection of oesophagitis. For example, the invention may be applied to the detection of eosinophilic oesophagitis. For example, the invention may be applied to detection of lymphocytic gastritis. For example, the invention may be applied to the detection of infections, since infections may be regarded as inflammatory conditions.

It is an advantage of the invention that a single stain such as H & E stain can be used to detect each of the diverse foreign cell types of interest.

As discussed below the term "foreign" means cells which are abnormally located in the tissue being assayed.
Foreign cell types according to the invention include fungal cells such as Candida *albicans*, bacterial cells such as *Helicobacter pylori*, inappropriate white blood cells such as lymphocytes, neutrophils, leukocytes or eosinophils.

As used herein the term "foreign" does not necessarily mean foreign to the subject. Instead, the term "foreign" means cells which should not normally be found in the upper gastrointestinal tract of a healthy subject. For example, a leukocyte or a neutrophil or a eosinophil would not be foreign (alien or non-host) in the sense of not belonging to the subject being investigated but would be regarded as "foreign" since those cells would not normally be found in the tissues of the upper gastrointestinal tract, such as in the epithelium layer of said tissues, of a healthy subject. Therefore, the term "foreign" means cells which are not normally found in the tissue being assayed, more suitably which are not normally found in the epithelium layer of said tissue.

Moreover, there are a very small number of cells which may be non-host cells i.e. cells not belonging to the subject being investigated but would nevertheless not be regarded as "foreign" because they are cells of commensal organisms. Thus commensal organisms are not regarded as "foreign". An example of a non-host cell which is commensal (and therefore not "foreign") is an actinomyces cell. Actinomyces bacterial cells are known to occur naturally in the tonsil. As discussed herein, *H. pylori* is suitably diagnosed by the stain or antibody. If a bacterial cell is observed but which stains negative (i.e. is not *H.pylori*) then this would be assumed to be commensal.

In more detail in the context of the present invention, foreign cells may comprise pathogens such as fungal cells or bacterial cells.

Foreign cells may comprise infectious cells such as fungal cells or bacterial cells. Foreign cells may comprise a subject's own inflammatory cells.
Foreign cells may comprise a subject's own cells (such as a subject's own white blood cells), such as leukocytes, neutrophils or eosinophils, but which are inappropriately located in a tissue being examined, such as in the epithelium layer of said tissue. More suitably foreign cells may comprise neutrophils or eosinophils. Most suitably foreign cells may comprise eosinophils.
Foreign cells may comprise a subject's own cells infected with a virus such as Herpes virus.

When applied to the detection of, or diagnosis of, or gathering information useful in aiding the diagnosis of, infection(s) then the "foreign" cell may be an alien cell and/or may be a non-host cell, such as for example a C.albicans cell, a H.pylori cell, or an *Aspergillus* cell.

Observing fungal cells indicates a diagnosis of fungal infection. Observing C.albicans cells indicates a diagnosis of C.albicans infection. Observing Aspergillus cells indicates a diagnosis of *Aspergillus* infection.

Observing bacterial cells indicates a diagnosis of bacterial infection. Observing H.pylori cells indicates a diagnosis of *H.pylori* infection.

Observing host cells infected with virus such as *Herpes* virus indicates a diagnosis of viral infection such as Herpes virus infection.

Observing leukocytes indicates a diagnosis of oesophagitis.
Observing neutrophils indicates a diagnosis of oesophagitis.
Observing leukocytes and neutrophils indicates a diagnosis of oesophagitis.
Observing lymphocytes indicates a diagnosis of lymphocytic gastritis. In particular, observing lymphocytes in or near glandular epithelial cells (e.g. stomach cells) indicates a diagnosis of lymphocytic gastritis (inflammation of the stomach). More suitably observing >1 lymphocyte per 5 epithelial cells indicates a diagnosis of lymphocytic gastritis more suitably observing >25 lymphocytes per 100 glandular epithelial cells indicates a diagnosis of lymphocytic gastritis.

Observing eosinophils indicates a diagnosis of eosinophilic oesophagitis.
More suitably observing leukocytes and eosinophils indicates a diagnosis of eosinophilic oesophagitis.

More suitably observing neutrophils and eosinophils indicates a diagnosis of eosinophilic oesophagitis.
Most suitably observing leukocytes and neutrophils and eosinophils indicates a diagnosis of eosinophilic oesophagitis.

Foreign cells may comprise a subject's own cells such as macrophages. Observation of macrophage indicates that the condition may be a chronic condition. Observation of macrophages does not itself lead to a specific diagnosis, but rather aids or adds detail to diagnosis of a particular condition; observation of macrophages indicates that a condition is chronic. More suitably observation of macrophage in combination with a diagnosis of oesophagitis indicates a further diagnosis of chronic oesophagitis. Macrophage are identified using conventional stain such as H&E stain as for other foreign cells discussed.

Advantageously the conditions being diagnosed are benign. Cell collection devices of the type described herein have previously only been used for malignant conditions. Thus, suitably the invention is not applied to Barrett's oesophagus. Suitably the foreign cells are not Barrett's oesophagus cells.
Thus, suitably the invention is not applied to Barrett's related abnormalities. Suitably the foreign cells are not cells of Barrett's related abnormalities.
Thus, suitably the invention is not applied to dysplasia. Suitably the foreign cells are not dysplastic cells.
Thus, suitably the invention is not applied to adenocarcinoma. Suitably the foreign cells are not adenocarcinoma cells.
Thus, suitably the invention is not applied to non-squamous cells. Suitably the foreign cells are not non-squamous cells.
Thus, suitably the invention is not applied to squamous carcinoma. Suitably the foreign cells are not squamous carcinoma cells.

Suitably the invention is used to detect one or more inflammatory condition(s). Previously cell collection devices as described herein have only been used for non-inflammatory conditions such as malignancies.

Suitably each of the disorders diagnosed as described herein can be detected by a simple H & E stain.

It is an advantage of the invention that in a preferred embodiment a simple method is provided involving one cell collection device, one protocol for cell preparation, and one simple H & E stain which together form a cohesive method for detection of a range of foreign cells.

Some "foreign" cells are occasionally referred to as infiltrators, or as inflammatory infiltrators. By contrast, Barrett's cells grow from, or change from, existing cell types which are normally resident in tissues such as the oesophagus. Barrett's cells are not infiltrator cells. Neutrophils or other foreign cells which would not normally be found in the oesophagus (such as in the epithelium layer of the oesophagus) in a healthy individual are found in that tissue (or in the epithelium layer of that tissue) in certain pathological disorders. They migrate or infiltrate the tissue (such as in the epithelium layer of said tissue). For example, H.pylori can infiltrate and be introduced from the air. For example, Candida albicans can infiltrate and be introduced from food or drink or other swallowed substances. Aspergillus can infiltrate and be introduced from food or drink or other swallowed substances. Herpes virus can infiltrate and be introduced from food or drink or other swallowed substances.

### Location of "foreign" cells

It is an advantage of the invention that cells from the epithelium layer are sampled. Suitably the sampling techniques described herein have the advantage of sampling cells from the epithelium layer. Typically tissue structure is disrupted to a degree by such sampling.
Thus epithelial cells appear as clumps in the sample.
Such clumps may be described as groups or as clusters.
A clump contains enough cells to see that they are closely associated.

Thus in some embodiments the location of the foreign cell may be taken into account. By location is meant the immediate cellular environment of the foreign cell.
Suitably the foreign cell is within epithelium.
By within epithelium is meant within a cluster/clump/group of epithelial cells.
By within is meant that if a boundary was drawn around the outermost extremity of a cluster of epithelial cells observed in a sample, the foreign cell could be observed to lie within this boundary i.e. within the epithelial cells.

Suitably if bacteria are within the epithelium (epithelial cells) then diagnosis is confirmed. Moreover, if inflammatory cells are seen with the epithelium and bacteria then diagnosis is confirmed.

Suitably if virus infected host cells are within the epithelium (epithelial cells) then diagnosis of viral infection is confirmed.

Candida may be diagnosed when fungal hyphae are observed, in particular when within the epithelium diagnosis is confirmed. Aspergillus may be diagnosed when fungal hyphae are observed, in particular when within the epithelium diagnosis is confirmed. Commensal or other pathogenic fungi are very unusual. Therefore observation of hyphae, in particular within the epithelium, infers diagnosis of Aspergillus or Candida is confirmed. Candida is more common than Aspergillus, and so in the absence of further indications towards which fungal species is present then there is a greater likelihood of the fungal infection being Candida infection. Criteria for distinguishing these fungi are well known. In case any further guidance is required, this is discussed in more detail below.

Suitably if neutrophils are observed within glandular or squamous epithelial cells, diagnosis is confirmed.

Suitably if lymphocytes are observed within glandular epithelial cells, this infers that a diagnosis of lymphocytic gastritis is confirmed.

### Normal Cells

Cells which are expected to be seen (and are therefore not indicative of a disorder and are not regarded as foreign) include squamous cells, glandular cells from the stomach, respiratory type glandular epithelium.

Squamous epithelium is derived from the oesophagus.
Glandular epithelium is derived from the stomach.

It is also normal to observe granular debris. Debris does not comprise cells. Debris appears as grainy material with no cells.

Debris appears as granular material. Debris is acellular. Debris suitably shows an absence of intact cells. Debris is typically made up of tiny bacterial structures which has a speckled appearance. In case any further guidance is needed, reference is made to the attached drawings.

### Detection and Stains

Suitably detection of foreign cells is by observation of the collected cells.

Suitably the cells are stained before observation. Suitably the cells are stained using haematoxylin and eosin (H&E) stain. This has the advantage of rendering the cells easily distinguished from one another according to conventional and long established histology.

Standard histology / cytology is used to tell the cells apart.

In case any further guidance is needed, reference is made to standard text books in this area such as Diagnostic Cytopathology by Winifred Gray 2nd edition. In addition, or alternatively, text books such as Gastrointestinal Pathology An Atlas and Textbook by Cecilia M. Fenoglio-Preiser, Amy E. Noffsinger, Grant N. Stemmermann, Patrick E. Lantz, Peter G. Isaacson Third edition may be used. These texts show the characteristics of the cell types mentioned herein. Any other conventional cytology/histology guides may be used if required.

### Haematoxylin and Eosin (H&E)

The haematoxylin and eosin stain uses two separate dyes, one staining the nucleus and the other staining the cytoplasm and connective tissue. Haematoxylin is a dark purplish dye that will stain the chromatin (nuclear material) within the nucleus, leaving it a deep purplish-blue colour. Eosin is an orangish-pink to red dye that stains the cytoplasmic material including connective tissue and collagen, and leaves an orange-pink counterstain. This counterstain acts as a sharp contrast to the purplish-blue nuclear stain of the nucleus, and helps identify other entities in the tissues such as cell membrane (border), red blood cells, and fluid.

The staining process involves hydration of the sample (if necessary}; staining with the nuclear dye (hematoxylin) and rinsing, then staining with the counterstain (eosin}. They are then rinsed, and if necessary dehydrated (e.g. treated with water, then alcohol and then xylene), and prepared for observation e.g. by addition of coverslips.

### Progressive/Regressive Staining

There are two methods for performing the H&E stain: Progressive in which the slides are placed in haematoxylin then rinsed and placed in eosin; and the regressive method in which the slides are placed in a stronger type of haematoxylin, then differentiated in acid alcohol to take the haematoxylin back out of everything except the nucleus, and then placed in eosin. In both types of staining, a bluing solution (Scott's Tap Water or ammonia water) is optionally used to cause the nucleus to turn a deep purplish blue color.

In progressive staining, a milder form of haematoxylin is used that will only stain the nucleus of the cell and cause the nuclear material to turn a deeper blue when rinsed in water. With this method the technician can simply stain, rinse and move on to the next step. Its advantage is simplicity, fewer steps, and avoids the possibility of over/under differentiation in acid alcohols. The level or colour of staining is standardized and consistent. Progressive staining has the advantage of easier automation.

Haematoxylin products for progressive staining are commercially available such as from Sigma Inc. (Sigma Aldrich) and include: Gill's 1, Gill's 2, Gill's 3, and Mayer's haematoxylin. The difference in the three Gill stains is the haematoxylin strength. Gill's 1 is used primarily for cytology staining where a weaker haematoxylin is adequate because you are staining individual cells from a fluid suspension, not tissue. Gill's 2 and 3 are stronger and generally used for histology staining. They are developed for tissue structure. The choice of whether to use Gill's 2 or 3 is a matter of preference for the skilled worker.

In regressive staining, a stronger form of haematoxylin is used called Harris haematoxylin. Harris haematoxylin will stain everything on the slide and hold fast to the tissue when rinsed. Therefore after staining and rinsing with water, the next step is to differentiate or take out the excess haematoxylin from everything except the nucleus. The slides are agitated in a mild acid alcohol solution that slowly removes the excess haematoxylin. After differentiating the slides are rinsed and placed in a bluing solution (Scott's Tap Water or ammonia water), which will cause the nucleus to turn a deep purplish blue colour.

Haematoxylin products for regressive staining are commercially available such as from Sigma Inc. (Sigma Aldrich) and include Harris haematoxylin.

After haematoxylin staining the samples are rinsed, and stained in eosin. If necessary, they may be dehydrated with graded strengths of alcohols, cleared in xylene and finally prepared for observation e.g. with coverslips and/or permanent mounting media.

Eosin products are commercially available such as from Sigma Inc. (Sigma Aldrich) and include Eosin Y, Eosin Y Alcoholic, and Eosin Y with Phloxine. Similar to the three types of Gill's stain, the eosins are differentiated by their strength and depth in colour. Eosin Y is the weakest of the three and gives a pink stain to the cytoplasm and collagen. Eosin Y Alcoholic is a stronger stain and gives a more brilliant orangish red colour due to its alcohol ingredient. Eosin Y with Phloxine is the strongest stain and has an overwhelmingly red colour due to the addition of phloxine. While the selection of eosin is a matter for the skilled worker, Eosin Y with Phloxine is generally considered too red for standard histology. Thus suitably the eosin used is Eosin Y Alcoholic.

It is an advantage of haematoxylin and eosin (H&E) stain that use of molecular markers for specific cell types can be avoided.

It is an advantage of haematoxylin and eosin (H&E) stain that cells from different sources or species (e.g. bacteria, fungi, higher eukaryotes) may be positively observed and/or easily distinguished by use of only a single stain. This has the advantage of avoiding the need for different molecular markers for different organisms or cell types.

### Giemsa stain

Optionally Giemsa stain may also be applied to the sample. This is advantageous in identification of eosinophils and/or H.pylori*.* This is especially advantageous in identification of *H.pylori.* Thus Giemsa is most suitably used to aid detection of H.pylori*.*

Giemsa stain may include variants of Giemsa stain such as Modified Grenwalt Giemsa (MGG).

Giemsa's solution is a mixture of methylene blue, eosin, and azure B. The stain is usually prepared from commercially available Giemsa powder.

Typically, the sample is fixed in pure methanol for 30 seconds, by immersing it or by putting a few drops of methanol onto it. The sample is then immersed in a freshly prepared 5% Giemsa stain solution for 20-30 minutes, or 5-10 minutes in 10% solution, then flushed with tap water and left to dry.

Giemsa stain can be performed on the same slide and at the same time as H&E stain.

### Urease Enzyme

A rapid urease test, also known as the CLO test (Campylobacter-like organism test), is a rapid test for diagnosis of Helicobacter pylori. The basis of the test is the ability of *H*. *pylori* to secrete the urease enzyme, which catalyzes the conversion of urea to ammonia and bicarbonate.
The sample is placed into a medium containing urea and an indicator such as phenol red. The urease produced by *H. pylori* hydrolyzes urea to ammonia, which raises the pH of the medium, and changes the color of the specimen from yellow (NEGATIVE) to red (POSITIVE).

Thus suitably the method of the invention comprises the further step of conducting a urease test. A positive result of the urease test indicates a diagnosis of *H*. *pylori.*

### Antibodies for Detection of H.pylori

Optionally an antibody test for *H. pylori* may also be applied to the sample.
For example, Leica H. pylori monoclonal antibody may be used with a Bond Max™ autostainer following manufacturer's instructions.

The antibody may be any suitable one known in the art, for example HPP (NCL-HPp) polyclonal, HPYLORI-L-CE-S (NCL-L-Hpylori), or HPYLORI-L-CE (NCL-L-Hpylori), all of which are commercially available from Leica Biosystems (Venture Park Stirling Way, Bretton Peterborough, PE3 8YD United Kingdom).

### Detection of Herpes

Herpes virus (e.g. Herpes simplex virus, HSV) infection can be identified from the appearance of the foreign cells (which in this embodiment are the host cells infected with the virus). The infected squamous cells show multinucleation with multiple moulded nuclei demonstrating a homogenized ground glass appearance.

In addition, if further confirmation is desired, a nucleic acid test such as a PCR test may be carried out to check the virus or viral type present.

### Aspergillus

*Aspergillus* cells comprise septate hyphae 3-4 micrometers in width and demonstrating dichotomous branching at 45 degree angles.
Fruiting heads may also be seen.

### Further Applications

In another aspect, the invention provides a kit comprising a swallowable device comprising abrasive material capable of collecting cells from the surface of the oesophagus, together with printed instructions for its use in detection of foreign cells.

In another aspect, the invention provides a kit comprising a swallowable device comprising abrasive material capable of collecting cells from the surface of the oesophagus, together with printed instructions for its use in detection of an inflammatory condition.

### Combination Applications

In one embodiment the invention relates to a combination method for Barrett's oesophagus and/or Barrett's associated dysplasia such as squamous cell dysplasia, which are diagnosed using specific biomarkers as known in the art, together with detection of foreign cells/diagnosis of benign conditions as described herein. These conditions account for the vast majority of oesophageal diagnoses that would be evaluated in an endoscopy [3].

Thus the invention relates to a method as described above comprising the further step of assaying the cells for a non-squamous cellular marker, wherein detection of such a marker indicates increased likelihood of the presence of Barrett's or Barrett's associated dysplasia.

Suitably the non-squamous cellular marker is a marker of cellular proliferation.

Suitably the non-squamous cellular marker is a marker of columnar cells.

Suitably the marker is selected from the group consisting of brush border proteins such as villin or moesin, mucin genes, brush border enzymes such as alkaline phosphatase, homeobox genes such as Cdx1 and/or Cdx2, cytokeratins such as CK8/18 for columnar cells, or any marker known to be differentially expressed in Barrett's versus normal oesophageal surface cells.

Suitably the marker is selected from the group consisting of proliferation markers such as Ki67 and Mcm proteins, proliferation and DNA damage markers such as PCNA, cyclins such as cyclin D and/or cyclin A, abnormal p53, loss of p16, aneuploidy or any marker known to correlate with the degree of dysplasia.

Suitably the marker is Mcm2 or Cyclin A. Suitably the marker is Cyclin A. Suitably both Mcm2 and Cyclin A are assayed.

In another aspect, the invention relates to a method as described above further comprising analysing the chromosomal composition of the cells, wherein detection of abnormal karyotype indicates an increased likelihood of dysplasia.

In another aspect, the invention relates to a method further comprising analysing the p53 status of the cells, wherein detection of abnormal p53 status indicates an increased likelihood of dysplasia.

Preferably the method of the invention is conducted in vitro*.*

In another aspect, the invention provides a kit as described above further comprising a local anaesthetic. Preferably said local anaesthetic is a spray or lozenge, preferably a spray.

In another aspect, the invention provides a kit as described above further comprising a container for receiving said swallowable device after withdrawal, said container having a quantity of preservative fluid therein. Preferably the container is a watertight container. Preferably the preservative fluid is a cell preparation fluid. Preferably said fluid is thin preparation fluid for production of slides for examination of the sampled cells.

In another aspect, the invention provides a kit as described above wherein said device comprises a capsule sponge.

In another aspect, the invention provides a kit as described above wherein said swallowable device comprises withdrawal means such as string.

In another aspect, the invention provides a kit as described above further comprising a device for severing said withdrawal means. Preferably said device comprises a blade or scissors.

In another aspect, the invention provides a kit as described above further comprising a container for administering drinkable fluid, such as water, to the subject.

In another aspect, the invention provides a kit as described above further comprising gloves. These advantageously protect the sample from contamination upon withdrawal of the device.

In another aspect, the invention provides a kit comprising a swallowable device comprising abrasive material capable of collecting cells from the surface of the oesophagus, together with printed instructions for its use in detection of benign conditions, suitably benign conditions in combination with Barrett's oesophagus or Barrett's associated dysplasia. Preferably said device comprises a capsule sponge.

Preferably said kit further comprises reagent for use in the detection of a non-squamous cellular marker. Preferably said non-squamous cellular marker is a marker of cellular proliferation. Preferably the non-squamous cellular marker is a marker of columnar cells.

In another aspect, the invention provides a kit as described above further comprising reagents for use in the detection of at least one marker selected from the group consisting of brush border proteins such as villin or moesin, mucin genes, brush border enzymes such as alkaline phosphatase, homeobox genes such as Cdx1 and/or Cdx2, cytokeratins such as CK8/18 for columnar cells, or any marker known to be differentially expressed in Barrett's versus normal oesophageal surface cells.

In another aspect, the invention provides a kit as described above further comprising reagents for use in the detection of at least one marker selected from the group consisting of proliferation markers such as Ki67 and Mcm proteins, proliferation and DNA damage markers such as PCNA, cyclins such as cyclin D and/or cyclin A, abnormal p53, loss of p16, aneuploidy or any marker known to correlate with the degree of dysplasia. Preferably said marker is Cyclin A.

Preferably said marker is a lectin.

In another aspect, the invention provides a kit further comprising a watertight container and preservative fluid. Preferably said fluid is for liquid based cytology, preferably said fluid is commercially available thin preparation fluid for production of slides for examination of the sampled cells.

Suitably the marker is Trefoil factor 3 (TFF3) wherein the presence of said TFF3 is indicative of said subject having Barrett's oesophagus or Barrett's associated dysplasia.

### Brief Description of the Figures

Figure 1 shows representative immunohistochemistry for *H. pylori* in a Cytosponge specimen.
Figure 2 shows representative H&E staining of Candida.
Figures 3 to 10 show photographs of cells.

The invention is now described by way of example. These examples are intended to be illustrative, and are not intended to limit the appended claims.

### Examples

In these examples, non-endoscopic diagnostic tests for oesophageal diseases and H.pylori are demonstrated. In these examples, the cell collection device is the cytosponge.

### Example 1:

We demonstrate:
1) determination whether inflammatory cells including eosinophils, Candida and *H.pylori* could be detected on the Cytosponge sample;
2) use of data from the BEST1 cohort study of over 500 individuals to obtain preliminary data on the diagnostic yield for these conditions using the Cytosponge compared to endoscopy.

### Study design

A prospective cohort study of the Cytosponge was undertaken in 12 UK general practices. Endoscopy was used as the gold standard diagnostic test for comparison and the primary purpose was to determine our ability to diagnose Barrett's oesophagus as previously described (BMJ 2010). The study was approved by Cambridge Regional Ethics Committee. Eligible patients were identified by searching the prescribing database of 12 primary care practices for individuals aged 50 to 70 years with a previous prescription for an acid-suppressant medication (H2 receptor antagonist or proton pump inhibitor) for more than three months over the past five years. Participants with: a previous diagnosis of Barrett's oesophagus; gastroscopy examination within the past one year; dysphagia; known portal hypertension; pharmacological or pathophysiological abnormality of coagulation; significant physical or psychological co-morbidity making them unsuitable for gastroscopy and/or inability to provide informed consent, were excluded. The number of surgeries included was based on previous primary care based studies which reported that 16·3% of contacted patients with reflux symptoms accepted to undergo endoscopy [6]. Recruitment continued until more than 500 individuals had participated.

### Study procedures

We carry out a method for detecting a foreign cell in a tissue of the upper intestinal tract.

The first step is (a) providing a sample of cells from the epithelium layer of the tissue of interest:
Following written informed consent the cell sampling was performed using a Cytosponge by a research nurse or research fellow in the primary care setting. The Cytosponge is an ingestible gelatine capsule containing a compressed mesh attached to a string which was approved by the MHRA for the purposes of this trial [7]. As previously described the capsule, swallowed with glass of water, dissolves in the proximal stomach to release a spherical mesh of 3 cm diameter. Five minutes after ingestion, the expanded mesh is withdrawn by pulling on the string to collect a cytological specimen.
All those who swallowed the Cytosponge were invited to attend for a gastroscopy within 3 weeks of the screening test procedure. The gastroscopies were performed at a single specialist unit by one of three endoscopists who adhered to a strict diagnostic protocol. Any endoscopic findings were recorded and photographed and biopsies taken according to standard clinical practice guidelines. Oesophagitis was recorded according to the Savary Miller classification. Any plaques suspicious for *Candida* infection were noted.

The next step is (b) preparing the cells into a cohesive cluster:

### Cytosponge sample collection and processing

The specimens were immediately placed into Surepath preservative fluid (Gift from BD Diagnostics, Durham, NC, USA) and kept at 4°C until they were processed to paraffin blocks as described previously [7]:
Samples were left in preservative solution for a minimum of 1 h. The samples were incubated for 30 min in Cytolyt® solution (Cytyc Corporation, Crowley, UK), washed twice in PBS and pelleted at 250xg for 5 min. The resulting pellet was resuspended in 500ul of plasma and thrombin (Diagnostic reagents, Oxford, UK) was then added in 10ul increments until a clot formed. The clot was then placed in formalin for 24 h prior to processing into a paraffin block. The entire sample was cut in 5 um sections to provide 20 slides.

The next step was (c) staining cells from the cohesive cluster using haematoxylin and eosin (H&E) stain; this was done according to standard methods.

The next step was (d) observing the stained cells:
All reporting was performed by an expert gastrointestinal cytopathologist (MO'D). The pathologist assessing the diagnostic biopsy was blinded to the Cytosponge result.

The next step was (e) inferring the presence or absence of foreign cell(s) from the observations of (d).

### Example 2: Pathological diagnosis

We demonstrate a method for aiding the diagnosis of an inflammatory condition of the upper intestinal tract in a subject, said method comprising detecting a foreign cell according to example 1.

Detection of foreign cell(s) in step (e) was used to infer that the subject has an inflammatory condition of the upper intestinal tract.

In addition, the foreign cells detected were classified and this was used to infer the type of disorder based on the type(s) of cell(s) observed.

More specifically,
if the foreign cell is a leukocyte, it is inferred that the subject has oesophagitis;
if the foreign cell is a neutrophil, it is inferred that the subject has oesophagitis;
if the foreign cell is a eosinophil, it is inferred that the subject has eosinophilic oesophagitis;
if the foreign cell is *H.pylori,* it is inferred that the subject has *H.pylori* infection; and
if the foreign cell is *C.albicans,* it is inferred that the subject has *C.albicans* infection.

Biopsies were collected from each patient for Helicobacter pylori testing using a CLO test. On both biopsies and Cytosponge specimens stained with hematoxylin and eosin, acute and chronic inflammation were assessed using standard histopathological criteria. Presence of eosinophils were specifically noted and quantified. The presence of Candida was documented. Immunostaining for *H. pylori,* using the Leica H. pylori monoclonal antibody, was performed using a Bond Max™ autostainer following manufacturer's instructions. Cytosponges sections were scored as displaying presence or absence of *H*. *pylori* in a binary fashion. The Histopathologist was unaware of the endoscopic diagnosis in all cases.

### 1) Oesophagitis

17/504 patients had evidence of clinically significant endoscopic oesophagitis (Grades 2, 3 and 4) on their endoscopy. Inflammatory cells were identified in Cytosponge samples for 10/13 patients with Grade 2 oesophagitis and all 4 patients with grades 3 and 4 disease.

**Table 1: inflammation in Cytosponge samples compared to pathological diagnosis on endoscopic biopsies**

| Endoscopic oesophagitis | Grade 2 | Grade 3/4 |
|---|---|---|
| **Total** | **13** | **4** |
| Chronic | 2 | 1 |
| Acute | 8 | 3 |
| Chronic or acute | 8 | 4 |
| Chronic and acute | 2 | 0 |

### 2) Eosinophilic Oesophagitis

Occasional eosinophils were seen in 4 cases with oesophagitis in keeping with this diagnosis. In 2 cases there was a dense eosinophilic infiltrate. One of these cases was the same as the only clinical diagnosis of eosinophilic oesophagitis made in the study cohort. The other case is suspicious for this diagnosis but confirmatory biopsies were not taken at the time of endoscopy, highlighting the possibility of the superiority of the Cytosponge due to make this diagnosis due to the larger surface area sampled.

### 3) Inflections - H.pylori:

This bacterium colonises columnar mucosa. Therefore the presence of gastric cardia cells in the sample was required to assess H.pylori. This is one of the measures that we use to assess whether or not the Cytosponge has gone beyond the gastro-oesophageal junction. If presence of columnar tissue was confirmed in the Cytosponge specimen, *H. pylori* was detected with a sensitivity and specificity of 100% compared to the gold standard CLO test (Table 2).

**Table 2: Accuracy of detection of H. pylori in Cytosponge specimen compared to the gold-standard CLO test.**

| | Positive | Negative |
|---|---|---|
| CLO Test | 7 | 13 |
| Cytosponge | 7 | 13 |

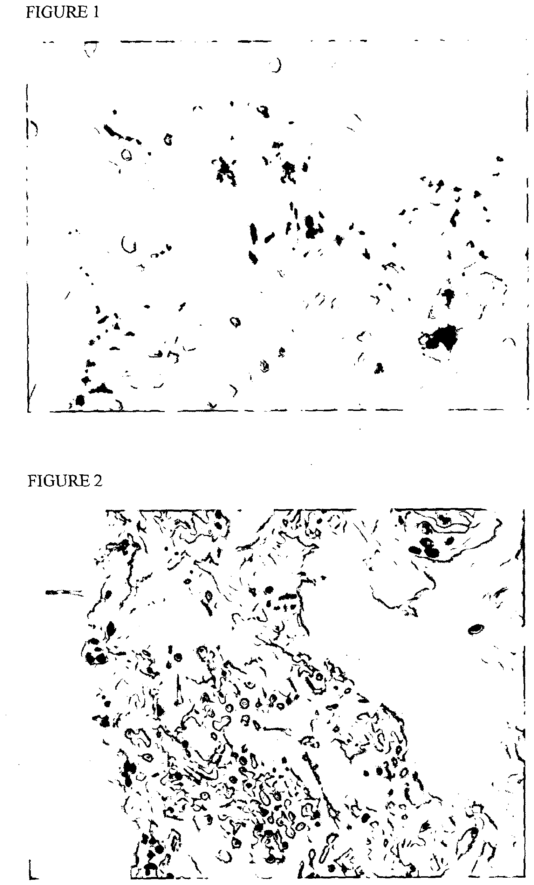

Figure 1 shows representative immunohistochemistry for *H. pylori* in a Cytosponge specimen

### 4) Infections - Candida

Candida infection was present in one case from the study cohort and this could be visualised on the Cytosponge as shown in Figure 2.

Thus we demonstrate that a histopathological assessment of cells collected from the upper intestinal tract using a cell collection device such as the cytosponge is able to diagnose common conditions affecting the oesophagus as well as the stomach (e.g. *H.pylori* infection).
This has industrial applicability as a simple diagnostic test for most common conditions affecting the oesophagus and proximal stomach which could be delivered in primary care thus bypassing the need for endoscopy which is invasive and costly.

In addition, optionally the assessment of cytokine levels such as IL13 and/or IL15 and/or the ligand CCL26 (eotaxin-3) might be used as a immunohistochemical marker or via rt-PCR for a more quantitative assessment eosinophilic infiltration.
Similarly for oesophagitis the level of inflammation could optionally be quantified by measuring CD45 (a pan-inflammatory cell marker).

### Example 3: Further Diagnosis of Benign Conditions

Samples comprised cells collected on a Cytosponge.

To add to the data set already presented in the above examples, Cytosponge samples collected from an independent set of patients were analysed. Samples from patients recruited to the BEST2 study (n=625) were reviewed by an expert histo-cytopathologist (Dr Maria O'Donovan).

Patients with Barrett's oesophagus or dyspepsia/reflux symptoms were recruited to the BEST2 study in 4 hospitals (Cambridge, Nottingham, University College London Hospital and Newcastle).

### Infectious diseases

As described herein, Candida (n=21) and Candida spores (n=14) could be identified in the Cytosponge samples. A total of 35 samples (5.6%) of the population were infected with either *Candida* or *Candida* spores.

Presence of *Helicobacter pylori* was examined in 432 patients from the cohort (according to whther it was clinically indicated at endoscopy to perform the gold standard clo test) and a total of 10 (2.3%) patients were positive for the bacteria.

In addition, we demonstrate detection and/or diagnosis of Herpes infection.
In addition, we demonstrate detection and/or diagnosis of *Aspergillus* infection.

### 5) Infections - Herpes

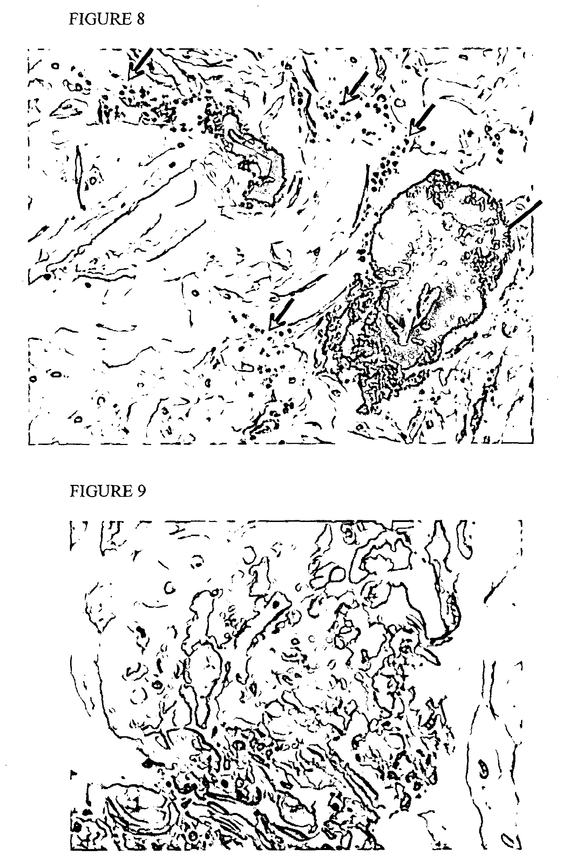

Herpes was detected as described herein.
We refer to Figure 9.
The squamous cells show multinucleation with multiple moulded nuclei demonstrating a homogenized ground glass appearance.
Thus herpes infection is detected according to the present invention.

### 6) Infections - Aspergillus

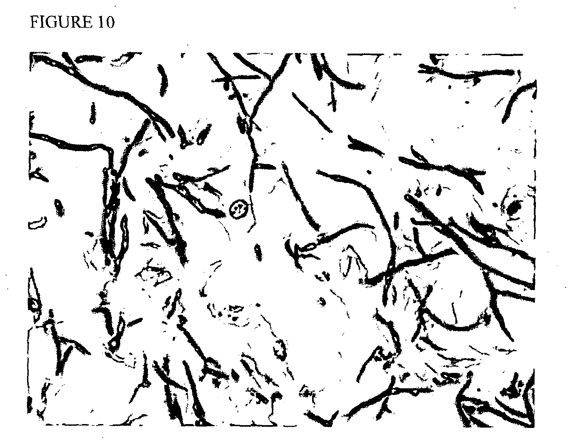

*Aspergillus* was detected as described herein.
We refer to Figure 10.
Septate hyphae 3-4 micrometers in width and demonstrating dichotomous branching at 45 degree angles are observed.
Thus Aspergillus infection is detected according to the present invention.

### Example 4: Observations

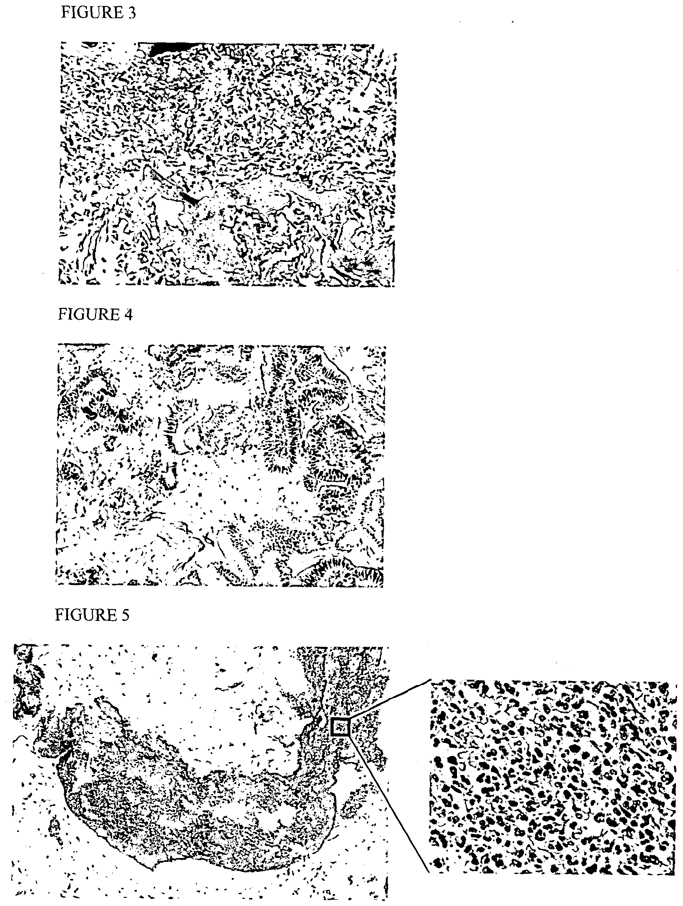

Figure 3 shows An example of squamous cells on the cytosponge
Figure 4 shows An example of glandular groups on the cytosponge
Figure 5 shows An example of a large group of neutrophils (inset = neutrophils)

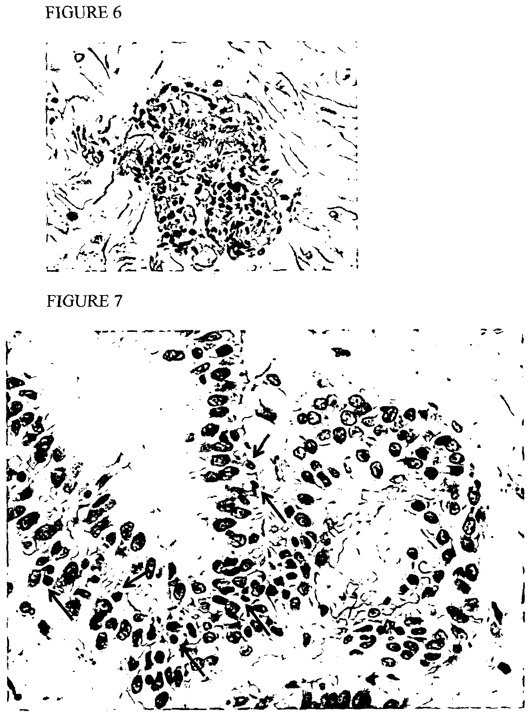

Figure 6 shows An example of a small group of neutrophils
Figure 7 shows An example of lymphocytic gastritis identified on the sponge; Arrows indicate examples of lymphocytes
Figure 8 shows Bacteria from tonsils with associated inflammatory cells; the green arrow (on the right, extending out of the photograph) shows Bacterial infection from the tonsils; Red arrows (internal to the photograph) indicate inflammatory cells
Figure 9: shows a representative picture of Herpes infection
Figure 10: shows a representative picture of Aspergillus infection

### References

1. Kadri, S., Lao-Sirieix, P.S., O'Donovan, M., Debiram, I., Das, M., Morris, H., Pharoah, P., Hardwick, R., Fitzgerald, R.C., A prospective, multicentre study of a novel non-endoscopic screening device for Barrett's oesophagus in primary care. British Medical Journal, 2010. **in press**.
2. Peters., C.J., et al., A clinically applicable four gene signature independently predicts survival in oesophageal and junctional adenocarcinoma. Gastroenterology, 2010. **Epub ahead of print**.
3. Warrell, D., T. Cox, and J. Firth, eds. Oxford Textbook of Medicine. Vol. 2. 2010, Oxford University Press.
4. Selgrad, M., et al., Clinical aspects of gastric cancer and Helicobacter pylori-screening, prevention, and treatment. Helicobacter, 2010. 15 Suppl 1: p. 40-5.
5. Garcia-Compean, D., et al., Prevalence of eosinophilic esophagitis in patients with refractory gastroesophageal reflux disease symptoms: A prospective study. Dig Liver Dis, 2011. 43(3): p. 204-8.
6. Corder, A.P., et al., Heartburn, oesophagitis and Barrett's oesophagus in self-medicating patients in general practice. Br J Clin Pract, 1996. 50(5): p. 245-8.
7. Lao-Sirieix, P., et al., Non-endoscopic screening biomarkers for Barrett's oesophagus: from microarray analysis to the clinic. Gut, 2009. 58(11): p. 1451-9.

Various modifications and variations of the described aspects and embodiments of the present invention will be apparent to those skilled in the art without departing from the scope of the present invention. Although the present invention has been described in connection with specific preferred embodiments, it should be understood that the invention as claimed should not be unduly limited to such specific embodiments. Indeed, various modifications of the described modes for carrying out the invention which are apparent to those skilled in the art are intended to be within the scope of the following claims.

## Claims

1. A method for aiding the diagnosis of an inflammatory condition of the upper gastrointestinal tract in a subject, said method comprising detecting a foreign cell in a tissue of the upper gastrointestinal tract by:
(a) preparing a sample of cells from an epithelium layer of the tissue of interest into a cohesive cluster, wherein said sample of cells consists of cells from a surface of the upper gastrointestinal tract collected by a swallowable device comprising abrasive material capable of collecting cells from a surface of an oesophagus;
(b) staining cells from the cohesive cluster using haematoxylin and eosin (H&E) stain;
(c) observing the stained cells; and
(d) inferring the presence or absence of foreign cell(s) from the observations of (c), wherein the foreign cell is selected from the group consisting of: leukocyte, neutrophil, eosinophil, lymphocyte, *H*.*pylori*, *C.albicans, Aspergillus,* and a cell infected with *Herpes* virus, and wherein detection of foreign cell(s) in step (d) infers that the subject has an inflammatory condition of the upper gastrointestinal tract.

2. A method according to claim 1 wherein the cohesive cluster is prepared by combining the cells with a sample of plasma and inducing said mixture to clot.

3. A method according to claim 1 wherein the cohesive cluster is prepared by combining the cells with agar or agarose.

4. A method according to any of claims 1 to 3 wherein detection of foreign cell(s) infers that the subject has a disorder of the upper gastrointestinal tract, said method further comprising classifying the foreign cell(s) observed in step (c), and inferring the type of disorder based on the type(s) of cell(s) observed.

5. A method according to claim 4 wherein;
if the foreign cell is a leukocyte, it is inferred that the subject has oesophagitis;
if the foreign cell is a neutrophil, it is inferred that the subject has oesophagitis;
if the foreign cell is a eosinophil, it is inferred that the subject has eosinophilic oesophagitis;
if the foreign cell is a lymphocyte, it is inferred that the subject has lymphocytic gastritis;
if the foreign cell is *H*.*pylori*, it is inferred that the subject has *H*.*pylori* infection; if the foreign cell is *C*.*albicans*, it is inferred that the subject has *C*.*albicans* infection;
if the foreign cell is *Aspergillus,* it is inferred that the subject has *Aspergillus* infection; and
if the foreign cell is a cell infected with *Herpes* virus, it is inferred that the subject has *Herpes* infection.

6. A method according to any preceding claim wherein said inflammatory condition is an infection.

7. A method according to claim 6 wherein said sample comprises cells from the gastric cardia.

8. A method according to any preceding claim wherein the tissue is oesophagus.

9. A method according to any preceding claim, further comprising performing a Giemsa stain.

10. A method according to any preceding claim, further comprising testing for urease activity.

11. A method according to any preceding claim, further comprising staining using an antibody reactive against *H*.*pylori*.

12. A method according to any of claims 1 to 11 comprising the further step of assaying the cells for a non-squamous cellular marker, wherein detection of such a marker indicates increased likelihood of the presence of Barrett's or Barrett's associated dysplasia, wherein the marker is selected from the group consisting of proliferation markers such as Ki67 and Mcm proteins, proliferation and DNA damage markers such as PCNA, cyclins such as cyclin D and/or cyclin A, abnormal p53, loss of p16, aneuploidy or any marker known to correlate with the degree of dysplasia.

## Patentansprüche

1. Verfahren zum Unterstützen der Diagnostik eines entzündlichen Zustands des oberen Magen-Darm-Trakts bei einem Subjekt, wobei das Verfahren das Feststellen einer fremden Zelle in einem Gewebe des oberen Magen-Darm-Trakt umfasst durch:
a) Herstellen einer Probe von Zellen aus einer Epithelschicht des Gewebes, das von Interesse ist, in einer zusammenhängenden Anhäufung, wobei die Probe von Zellen aus Zellen aus einer Oberfläche des oberen Magen-Darm-Trakts besteht, die durch eine verschluckbare Vorrichtung gesammelt werden, die abrasives Material umfasst, das in der Lage ist, Zellen von einer Oberfläche eines Ösophagus einzusammeln:
b) Färben der Zellen aus der zusammenhängenden Anhäufung unter Anwendung von Hämatoxylin- und Eosin- (H&E) Färbemittel;
c) Beobachten der gefärbten Zellen; und
d) Rückschließen auf das Vorliegen oder die Abwesenheit fremder Zelle(n) aus den Beobachtungen von (c), wobei die fremde Zelle aus der Gruppe ausgewählt wird bestehend aus: Leukozyt, Neutrophil, Eosinophil, Lymphozyt, H. pylori, C. albicans, Aspergillus und einer mit Herpes-Virus infizierter Zelle und wobei die die Feststellung der fremden Zelle(n) in Schritt (d) den Rückschluss ermöglicht, dass das Subjekt an einem entzündlichen Zustand des oberen Magen-Darm-Trakts leidet.

2. Verfahren nach Anspruch 1, wobei die zusammenhängende Anhäufung durch Kombinieren der Zellen mit einer Probe von Plasma und Induzieren, dass die Mischung koaguliert, hergestellt wird.

3. Verfahren nach Anspruch 1, wobei die zusammenhängende Anhäufung durch Kombinieren der Zellen mit Agar oder Agarose hergestellt wird.

4. Verfahren nach einem der Ansprüche 1 bis 3, wobei das Feststellen einer fremden Zelle/fremder Zellen den Rückschluss gestattet, dass das Subjekt an einer Erkrankung des oberen Magen-Darm-Trakts leidet, wobei das Verfahren ferner das Klassifizieren der fremden Zelle(n), die in Schritt (c) beobachtet worden sind, und den Rückschluss auf den Typ der Erkrankung auf der Basis des Typs/der Typen von beobachteten Zelle(n) umfasst.

5. Verfahren nach Anspruch 4, wobei:
wenn die fremde Zelle ein Leukozyt ist, der Rückschluss gezogen wird, dass das Subjekt an Ösophagitis leidet;
wenn die fremde Zelle ein Neutrophil ist, der Rückschluss gezogen wird, dass das Subjekt an Ösophagitis leidet;
wenn die fremde Zelle ein Eosinophil ist, der Rückschluss gezogen wird, dass das Subjekt an eosinophiler Ösophagitis leidet;
wenn die fremde Zelle ein Lymphozyt ist, der Rückschluss gezogen wird, dass das Subjekt an lymphozytischer Gastritis leidet;
wenn die fremde Zelle H. pylori ist, der Rückschluss gezogen wird, dass das Subjekt an einer H pylori-Infektion leidet;
wenn die fremde Zelle C. albicans ist, der Rückschluss gezogen wird, dass das Subjekt an einer C. albicans-Infektion leidet;
wenn die fremde Zelle Aspergillus ist, der Rückschluss gezogen wird, dass das Subjekt an einer Aspergillus-Infektion leidet; und
wenn die fremde Zelle eine mit dem Herpesvirus infizierte Zelle ist, der Rückschluss gezogen wird, dass das Subjekt an einer Herpesinfektion leidet.

6. Verfahren nach irgendeinem der vorhergehenden Ansprüche, wobei der entzündliche Zustand eine Infektion ist.

7. Verfahren nach Anspruch 6, wobei die Probe Zellen aus dem unteren Ösophagussphinkter umfasst.

8. Verfahren nach irgendeinem vorhergehenden Anspruch, wobei das Gewebe Ösophagusgewebe ist.

9. Verfahren nach irgendeinem vorhergehenden Anspruch, ferner das Ausführen einer Giemsa-Färbung umfassend.

10. Verfahren nach irgendeinem vorhergehenden Anspruch, ferner das Testen auf Ureaseaktivität umfassend.

11. Verfahren nach irgendeinem vorhergehenden Anspruch, ferner das Färben unter Anwendung eines gegen H. pylori reaktiven Antikörpers umfassend.

12. Verfahren nach irgendeinem der Ansprüche 1 bis 11, umfassend den weiteren Schritt des Untersuchens der Zellen auf einen nichtsquamösen Zellmarker, wobei das Feststellen eines derartigen Markers auf die erhöhte Wahrscheinlichkeit des Vorliegens von Barrett-Dysplasie oder mit Barret verbundener Dysplasie hinweist, wobei der Marker aus der Gruppe ausgewählt ist bestehend aus Proliferationsmarkern wie Ki67- und Mcm-Proteinen, Proliferations- und DNA-Schadenmarkers wie PCNA, Cyclinen wie Cyclin D und/oder Cyclin A, anormalem p53, Verlust an p16, Aneuploidie oder irgendeinem Marker, von dem bekannt ist, dass er mit dem Grad der Dysplasie korreliert.

## Revendications

1. Procédé permettant de favoriser le diagnostic d'un état inflammatoire des voies gastro-intestinales supérieures chez un sujet, ledit procédé comprenant la détection d'une cellule étrangère dans un tissu des voies gastro-intestinales supérieures par :
(a) préparation d'un échantillon de cellules issues d'une couche d'épithélium du tissu d'intérêt en une grappe cohésive, dans lequel ledit échantillon de cellules est constitué de cellules provenant d'une surface des voies gastro-intestinales supérieures recueillies par un dispositif avalable comprenant une matière abrasive capable de recueillir des cellules d'une surface d'un oesophage ;
(b) coloration de cellules de la grappe cohésive en utilisant un colorant d'hématoxyline et d'éosine (H&E) ;
(c) observation des cellules colorées ; et
(d) déduction de la présence ou de l'absence de cellule(s) étrangère(s) à partir des observations de (c), dans lequel la cellule étrangère est choisie dans le groupe constitué des suivantes : leucocyte, granulocyte neutrophile, éosinophile, lymphocyte, *H. pylori, C. Albicans,* Aspergillus et une cellule infectée par le virus *Herpes,* et dans lequel la détection de cellule(s) étrangère(s) à l'étape (d) implique que le sujet a un état inflammatoire des voies gastro-intestinales supérieures.

2. Procédé selon la revendication 1, dans lequel la grappe cohésive est préparée en combinant les cellules avec un échantillon de plasma et en induisant ledit mélange à se coaguler.

3. Procédé selon la revendication 1, dans lequel la grappe cohésive est préparée en combinant les cellules avec de l'agar ou de l'agarose.

4. Procédé selon l'une quelconque des revendications 1 à 3, dans lequel la détection de cellule(s) étrangère(s) implique que le sujet a un trouble des voies gastro-intestinales supérieures, ledit procédé comprenant en outre la classification de la cellule étrangère ou des cellules étrangères observées à l'étape (c) et la déduction du type de trouble sur la base du ou des types de cellule(s) observés.

5. Procédé selon la revendication 4, dans lequel :
si la cellule étrangère est un leucocyte, on en déduit que le sujet a une oesophagite ;
si la cellule étrangère est un granulocyte neutrophile, on en déduit que le sujet a une oesophagite ;
si la cellule étrangère est un éosinophile, on en déduit que le sujet a une oesophagite éosinophile ;
si la cellule étrangère est un lymphocyte, on en déduit que le sujet a une gastrite lymphocytaire ;
si la cellule étrangère est *H. pylori,* on en déduit que le sujet a une infection au *H. pylori ;*
si la cellule étrangère est *C. albicans,* on en déduit que le sujet a une infection au *C. albicans ;*
si la cellule étrangère est *Aspergillus,* on a déduit que le sujet a une infection à *l'Aspergillus ;* et
si la cellule étrangère est une cellule infectée par le virus *Herpes,* on en déduit que le sujet a une infection à l*'Herpes.*

6. Procédé selon l'une quelconque des revendications précédentes, dans lequel ledit état inflammatoire est une infection.

7. Procédé selon la revendication 6, dans lequel ledit échantillon comprend des cellules issues du cardia gastrique.

8. Procédé selon l'une quelconque des revendications précédentes, dans lequel le tissu est l'oesophage.

9. Procédé selon l'une quelconque des revendications précédentes, comprenant en outre la réalisation d'une coloration au Giemsa.

10. Procédé selon l'une quelconque des revendications précédentes, comprenant en outre des tests pour une activité d'uréase.

11. Procédé selon l'une quelconque des revendications précédentes, comprenant en outre une coloration en utilisant un anticorps réactif contre *H. pylori.*

12. Procédé selon l'une quelconque des revendications 1 à 11, comprenant l'autre étape d'analyse des cellules pour un marqueur cellulaire non squameux, dans lequel la détection d'un tel marqueur indique une augmentation vraisemblable de la présence de dysplasie de Barrett ou de la dysplasie associée de Barrett, dans lequel le marqueur est choisi dans le groupe constitué de marqueurs de prolifération tels que les protéines Ki67 et Mcm, des marqueurs de prolifération et d'endommagement d'ADN, tels que le PCNA, des cyclines telles que la cycline D et/ou la cycline A, d'une p53 anormale, d'une perte de p16, d'une aneuploïdie ou d'un marqueur quelconque connu pour corréler avec le degré de dysplasie.
